(19) Europäisches Patentamt
European Patent Office
Office européen des brevets

(11) **EP 3 515 401 B1**

(12) **EUROPEAN PATENT SPECIFICATION**


(45) Date of publication and mention of the grant of the patent:
**09.02.2022 Bulletin 2022/06**

(21) Application number: **17772582.7**

(22) Date of filing: **15.09.2017**

(51) International Patent Classification (IPC):
***A61K 8/34*** *(2006.01)* ***A61K 8/41*** *(2006.01)*
***A61Q 19/00*** *(2006.01)* ***A61K 8/04*** *(2006.01)*

(52) Cooperative Patent Classification (CPC):
**A61K 8/416; A61K 8/046; A61K 8/342; A61Q 19/00**

(86) International application number:
**PCT/US2017/051717**

(87) International publication number:
**WO 2018/057412 (29.03.2018 Gazette 2018/13)**


(54) **FOAM COMPOSITIONS, AEROSOL PRODUCTS, AND METHODS OF USING THE SAME**

SCHAUMZUSAMMENSETZUNGEN, AEROSOLPRODUKTE UND VERFAHREN UNTER EINSATZ DIESER

COMPOSITIONS DE MOUSSE, PRODUITS D'AÉROSOL, ET LEURS PROCÉDÉS D'UTILISATION


(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.09.2016 US 201662398580 P**

(43) Date of publication of application:
**31.07.2019 Bulletin 2019/31**



(73) Proprietor: **The Procter & Gamble Company**
**Cincinnati, OH 45202 (US)**

(72) Inventors:
- **SUNKEL, Jorge, Max**
**Cincinnati**
**Ohio 45202 (US)**
- **ZIMMERMAN, Dean, Arthur**
**Cincinnati**
**Ohio 45202 (US)**
- **MARSHALL, Larry, Wayne, Jr.**
**Cincinnati**
**Ohio 45202 (US)**

(74) Representative: **P&G Patent Belgium UK**
**N.V. Procter & Gamble Services Company S.A.**
**Temselaan 100**
**1853 Strombeek-Bever (BE)**



(56) References cited:
**EP-A1- 1 752 128** **WO-A1-2016/153946**
**JP-A- H08 253 409** **US-A1- 2003 053 961**
**US-A1- 2012 288 465**

- **Anonymous: "Hydrocarbon - Wikipedia", , 20 September 2017 (2017-09-20), XP055417470, Retrieved from the Internet: URL:https://en.wikipedia.org/wiki/Hydrocar bon [retrieved on 2017-10-19]**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



EP 3 515 401 B1

## Description

### TECHNICAL FIELD

The present disclosure generally relates to foam compositions, aerosol products, and methods of generating a foam composition that provides sensory benefits to consumers when applied to skin.

### BACKGROUND

There are many types of skin care products that are commercially available or otherwise known in the art, and there are many factors that can contribute to the purchase intent of a consumer when looking for such products. Critical among these factors are the sensory benefits that the skin care product can provide. As such, there is a consistent desire to develop new ways to deliver a positive sensory experience to consumers.

US 2003/053961 A1 discloses mousse-forming cosmetic compositions for the skin or hair, comprising quaternary ammonium agents.

Skin care products have often employed polymers as a way to manage rheological properties to promote performance benefits. However, some of these polymers are not optimized to provide the desired sensory benefits. For example, elevated polymer concentrations, relative to evaporating fluids, can thicken fluids that remain on the skin during product application and subsequent dry-down, resulting in tack, drag, stickiness, or other negative sensory aspects. Further, such negative aspects can continue after the dry-down phase as a result of sweating and humidity fluctuations.

Using a foam composition is one way to reduce or eliminate the use of polymers. For example, foams can use air to thicken a product in place of polymers. Thus, foams can convey a desired rich and creamy aesthetic while reducing or eliminating the negative sensory aspects associated with the use of polymers. Further, foams can easily absorb into the skin as they can rapidly break down into fluids. However, it is necessary for the foam to retain its structure long enough for the consumer to apply the product. If the foam collapses or disintegrates too quickly, the consumer will lose the associated pleasant sensory experience. Thus, there is a need to find a foamable composition that provides the desired sensory experience, while retaining its foam structure for a consumer acceptable period of time.

### SUMMARY

In accordance with the present invention, a method is provided, as in claim 1.

The present inventors have surprisingly found that in the case of the present invention, where a liquid foamable composition is formed of cationic and/or non-ionic surfactants together with fatty alcohol, use of a non-hydrocarbon propellant ensures the right quality of foam after dispensation. The combination of the cationic and/or non-ionic surfactant with fatty alcohol results in a foam with a structure that provides a desirable sensory feel on skin. The presence of cationic and/or nonionic surfactants in place of polymers ensures that the foam is not overly thick or tacky when applied to skin, while the fatty alcohol creates a lattice structure that holds the foam together.

In an embodiment, the foam has a polydispersity index of less than 2.5 immediately upon dispensation and for at least 60 seconds thereafter.

Use of a non-hydrocarbon propellant ensures that the structure of the foam is held for a period of time after dispensation.

To ensure the application process is satisfactory to a consumer, it is important that the foam retains its structure long enough after dispensation for the consumer to apply the product to their skin. In this respect, it would be undesirable if the foam were to collapse or if the composition were to separate prior to application of the product. Thus, preferably, at least 5 seconds after dispensation, the foam has a polydispersity index of less than 2.5.

The polydispersity index (PDI) is calculated as the standard deviation of the mean bubble area divided by the mean bubble area (SDMBA/MBA). Without being bound by theory, the fatty alcohols and surfactants present in the composition form a lamellar structure that stabilizes the foam structure. Use of a non-hydrocarbon propellant (or "compressed gas" as otherwise known), preferably carbon dioxide or nitrous oxide, permits this level of stability, whereas the use of a hydrocarbon gas propellant causes bubbles forming the foam to coalesce very rapidly, thus destabilizing the foam structure. For example, as set out in the examples, dimethyl ether has been shown to rapidly destabilize the foam structure resulting in a PDI of greater than 2.5 5 seconds after dispensation. Other hydrocarbon gases typically used in the industry but that are known to cause problems include dimethyl ether (DME), propane, isobutene and n-butane hydrocarbon mixtures.

PDI is measured vs other known techniques, such as foam density, as foam density is generally measured by dispensing the foam in a volumetric flask or similar to measure the foam volume and mass. As the bubble lamella structure starts breaking during coalescence, it causes the formation of some larger bubbles while smaller bubbles still remain (polydispersity). The top surface of the foam in the flask that is used to measure the volume for density calculations

does not drop rapidly as it is still being supported by some of the foam lamella beneath, and the weight remains the same. However, the structure of the foam has been compromised such that foam density is not a good way to track the structure of the foam post-dispensation. By contrast, PDI is based on comparing the bubble sizes of multiple frames of a video taken of the foam structure at very short intervals immediately after foam dispensing. From these screen shots, measurements are taken of the bubbles over time to provide a more accurate reflection of the changes in structure of the foam.

In an embodiment, the foam dispensed has a polydispersity index of between 1, 1.25 or 1.5 to 2, 2.25 or 2.5.

Preferably, the cosmetic foam has a foam density of 0.1 g/mL, 0.15 g/mL or 0.2 g/mL to 0.3 g/mL, 0.4 g/mL or 0.5 g/mL. The initial liquid foamable composition typically has a density of from about 0.9 g/mL to about 1.1 g/mL, and the density decreases as the liquid turns to foam. In embodiments, the liquid foamable composition is between 95% to 99.9% by weight of the cosmetic foam composition.

In the invention, the non-hydrocarbon gas is carbon dioxide or nitrous oxide, or a mixture thereof.

## BRIEF DESCRIPTION OF THE DRAWINGS

FIG. 1 depicts a side elevational view in partial section of an assembled valve mounted to a container according to one example.

FIG. 2 depicts a schematic cross-sectional view of an inner bag housed within a container according to another example.

FIG. 3 depicts a front view of the inner bag of FIG. 2.

FIGs. 4(a) and (b) show qualitatively the foam bubbles generated using a hydrocarbon propellant at 5 seconds and 60 seconds post dispensation (Comparative Example 1).

FIGs. 5(a), (b) and 6(a) and (b) show qualitatively the foam bubbles generated using two different non-hydrocarbon propellants and 5 seconds and 60 seconds post dispensation (Inventive Examples 1 and 2).

FIG. 7 is a graph showing the PDI for Comparative Example 1 and Inventive Examples 1 and 2.

## DETAILED DESCRIPTION

### I. Definitions

As used herein, the following terms shall have the meaning specified thereafter:

"Hydrocarbon propellants" are typically liquefied gases such as, for example, dimethyl ether (DME), propane, isobutene and n-butane hydrocarbon mixtures.

"Non-hydrocarbon propellants" are typically compressed gases such as, for example, carbon dioxide and nitrous oxide.

"Non-volatile," as it relates to at least fatty alcohols and silicones, can refer to having a boiling point at 1.0 atmospheres of about 260°C or greater, about 275°C or greater, or about 300°C or greater.

"Polymer" can refer to materials formed by polymerization of one type of monomer or formed by polymerization of two or more types of monomers (i.e., copolymers).

"Water soluble" can refer to being sufficiently soluble in water to form a solution that is substantially clear to a naked eye at a concentration of 0.1% in water (distilled or equivalent) at 25°C. The polymer can be sufficiently soluble to form a substantially clear solution at 0.5% concentration in water, and likely to form a substantially clear solution at 1.0% concentration in water.

II. Cosmetic Foam

Use of foams are desirable from a consumer perspective, as foams tend to provide a pleasant and light sensory experience while efficiently delivering actives into skin. In the present invention, the foamable composition is formed of a combination of a cationic and/or non-ionic surfactant and fatty alcohols. The present inventors have found that foams formed of these ingredients provide a stable structure, without compromising the sensory feel, in the way that polymers might. The present inventors have further found that non-hydrocarbon propellants used in conjunction with such a foamable composition provides a foam that remains stable for some time (at least 5 seconds) after dispensation. By comparison, use of a hydrocarbon, such as dimethyl ether, results in rapid destabilization of the foam following dispensation, as shown in the examples included herein.

Essential ingredients, as well as a non-exclusive list of optional ingredients, are described below.

Liquid Foamable Composition

A liquid foamable composition of the present invention includes a cationic and/or non-ionic surfactant and a fatty alcohol. Water and other optional ingredients (e.g., skin care actives, glycerin, a super-absorbent polymer) may also be included. Specific types and ranges for these components are described herein.

Surfactants

The liquid foamable composition may include from 0.05%, 0.1%, 0.5% or 1% to 2%, 3%, 4% or 5%, by weight of the liquid foamable composition, of surfactant. Surfactants of the present invention may be selected from the group consisting of: cationic surfactants, non-ionic surfactants and a mixture thereof. In a preferred embodiment, the surfactant comprises from 50%, 55%, 60% or 65% to 80%, 85%, 90%, 95% or 100%, by weight of the surfactant, of a cationic surfactant and from 0%, 5%, 10%, 15%, 20% to 35%, 40%, 45% to 50% of a nonionic surfactant.

Cationic Surfactants

Cationic surfactants suitable for use in the liquid foamable composition can include amino or quaternary ammonium moieties. Additional suitable cationic surfactants are disclosed in the following documents: M.C. Publishing Co., McCutcheon's, Detergents & Emulsifiers, (North American edition 1979); Schwartz, et al., Surface Active Agents, Their Chemistry and Technology, New York, Interscience Publishers, 1949; U.S. Patent No. 3,155,591, Hilfer, issued Nov. 3, 1964; U.S. Patent No. 3,929,678, Laughlin et al., issued Dec. 30, 1975; U.S. Patent No. 3,959,461, Bailey et al., issued May 25, 1976; and U.S. Patent No. 4,387,090, Bolich, Jr., issued Jun. 7, 1983.

Suitable quaternary ammonium compounds can include those of the general formula:

$$[NR1,R2,R3,R4\ ]^{+}\cdot X^{-}$$

wherein R1 to R4 can independently be an aliphatic group of from about 1 to about 22 carbon atoms or an aromatic, alkoxy, polyoxyalkylene, alkylamido, hydroxyalkyl, aryl, or alkylaryl group having from about 1 to about 22 carbon atoms; and $X^-$ can be a salt-forming anion, such as those selected from halogen (e.g., chloride, bromide, iodide), acetate, citrate, lactate, glycolate, phosphate nitrate, sulfate, and alkylsulfate radicals.

Such aliphatic groups can contain, in addition to carbon and hydrogen atoms, either linkages or other groups, such as amino groups. The longer-chain aliphatic groups (e.g., those of about 12 carbons, or higher) can be saturated or unsaturated. Mono-long alkyl quaternized ammonium salt cationic surfactants can include behenyl trimethyl ammonium salt, stearyl trimethyl ammonium salt, cetyl trimethyl ammonium salt, and hydrogenated tallow alkyl trimethyl ammonium salt. Di-long chain (e.g., di $C_{12}$-$C_{22}$, $C_{16}$-$C_{18}$, aliphatic, alkyl) and di-short chain (e.g., $C_1$-$C_3$ alkyl, $C_1$-$C_2$ alkyl) ammonium salts can also be employed. Other suitable quaternary ammonium salt useful as cationic surfactants are described in U.S. Patent No. 8,936,798.

Salts of primary, secondary, and tertiary fatty amines can also be suitable cationic surfactant materials. The alkyl groups of such amines can have from about 12 to about 22 carbon atoms, and may be substituted or unsubstituted. Such amines can include stearamidopropyl dimethylamine, behenylamidopropyl dimethylamine, diethyl amino ethyl stearamide, dimethyl stearamine, dimethyl soyamine, soyamine, myristyl amine, tridecyl amine, ethyl stearylamine, N-tallowpropane diamine, ethoxylated (5 moles E.O.) stearylamine, dihydroxy ethyl stearylamine, and arachidylbehenylamine. Suitable amine salts can include halogen, acetate, phosphate, nitrate, citrate, lactate, and alkyl sulfate salts. Such salts can include stearylamine hydrochloride, soyamine chloride, stearylamine formate, N-tallowpropane diamine dichloride, and stearamidopropyl dimethylamine citrate. Suitable cationic amine surfactants for the liquid foamable composition are disclosed in U.S. Patent No. 4,275,055, Nachtigal, et al., issued Jun. 23, 1981. In certain examples, suitable

cationic surfactants can include behenyl trimethyl ammonium chloride, stearyl ethylhexyldimonium methosulfate, dicetyldimonium chloride, ditallow dimethyl ammonium chloride, GENAMIN® CTAC (i.e., cetyl trimethyl ammonium chloride), esterquats (e.g., tetradecyl betainester chloride), diesterquats (e.g., dipalmitylethyl dimethyl ammonium chloride, ARMOCARE® VGH70 of Akzo, Germany), or a mixture of distearoylethyl hydroxyethylmonium methosulfate and Cetearyl Alcohol (DEHYQUART® F-75 of Henkel, Germany).

In certain examples, cationic surfactants (e.g., quaternary ammonium compounds) can be included at concentration levels from about 0.05% to about 5%, by weight, of the liquid foamable composition, and in certain examples, from about 1% to about 4%, by weight of the liquid foamable composition. Quaternary ammonium compounds may comprise one or more of behenyl trimethyl ammonium chloride, stearamidopropyl dimethylamine, behentrimonium methosulfate ("BTMS"), behenylamidopropyl dimethylamine, stearyl ethylhexyldimonium methosulfate, dicetyldimonium chloride, and ditallow dimethyl ammonium chloride.

Use of a cationic surfactant provides a foam that may provide a good sensory feel and remains stable at relatively low concentrations of surfactant. By comparison, if a low concentration of a non-ionic surfactant is used, although the liquid will be foamable, it may easily separate at elevated temperatures.

Nonionic Surfactants

Surfactants useful in the present invention may also be selected from nonionic surfactants. Among the nonionic surfactants that are useful herein are those that can be broadly defined as condensation products of long chain alcohols, e.g. C8-30 alcohols, with sugar or starch polymers, i.e., glycosides. These compounds can be represented by the formula $(S)_n$-O-R wherein S is a sugar moiety such as glucose, fructose, mannose, and galactose; n is an integer of from about 1 to about 1000, and R is a C8-30 alkyl group. Examples of long chain alcohols from which the alkyl group can be derived include decyl alcohol, cetyl alcohol, stearyl alcohol, lauryl alcohol, myristyl alcohol, oleyl alcohol, and the like. Preferred examples of these surfactants include those wherein S is a glucose moiety, R is a C8-20 alkyl group, and n is an integer of from about 1 to about 9. Commercially available examples of these surfactants include decyl polyglucoside (available as APG 325 CS from Henkel) and lauryl polyglucoside (available as APG 600 CS and 625 CS from Henkel).

Other useful nonionic surfactants include the condensation products of alkylene oxides with fatty acids (i.e. alkylene oxide esters of fatty acids). These materials have the general formula $RCO(X)_nOH$ wherein R is a C10-30 alkyl group, X is $-OCH_2CH_2-$ (i.e. derived from ethylene glycol or oxide) or $-OCH_2CHCH_3-$ (i.e. derived from propylene glycol or oxide), and n is an integer from about 6 to about 200. Other nonionic surfactants are the condensation products of alkylene oxides with 2 moles of fatty acids (i.e. alkylene oxide diesters of fatty acids). These materials have the general formula $RCO(X)_nOOCR$ wherein R is a C10-30 alkyl group, X is $-OCH_2CH_2-$(i.e. derived from ethylene glycol or oxide) or $-OCH_2CHCH_3-$(i.e. derived from propylene glycol or oxide), and n is an integer from about 6 to about 100. Other nonionic surfactants are the condensation products of alkylene oxides with fatty alcohols (i.e. alkylene oxide ethers of fatty alcohols). These materials have the general formula $R(X)_nOR'$ wherein R is a C10-30 alkyl group, X is $-OCH_2CH_2-$(i.e. derived from ethylene glycol or oxide) or $-OCH_2CHCH_3-$ (i.e. derived from propylene glycol or oxide), and n is an integer from about 6 to about 100 and R' is H or a C10-30 alkyl group. Still other nonionic surfactants are the condensation products of alkylene oxides with both fatty acids and fatty alcohols [i.e. wherein the polyalkylene oxide portion is esterified on one end with a fatty acid and etherified (i.e. connected via an ether linkage) on the other end with a fatty alcohol]. These materials have the general formula $RCO(X)_nOR'$ wherein R and R' are C10-30 alkyl groups, X is $-OCH_2CH_2$ (i.e. derived from ethylene glycol or oxide) or $-OCH_2CHCH_3-$ (derived from propylene glycol or oxide), and n is an integer from about 6 to about 100. Nonlimiting examples of these alkylene oxide derived nonionic surfactants include ceteth-6, ceteth-10, ceteth-12, ceteareth-6, ceteareth-10, ceteareth-12, steareth-6, steareth-10, steareth-12, PEG-6 stearate, PEG-10 stearate, PEG-100 stearate, PEG-12 stearate, PEG-20 glyceryl stearate, PEG-80 glyceryl tallowate, PEG-10 glyceryl stearate, PEG-30 glyceryl cocoate, PEG-80 glyceryl cocoate, PEG-200 glyceryl tallowate, PEG-8 dilaurate, PEG-10 distearate, and mixtures thereof.

Still other useful nonionic surfactants include polyhydroxy fatty acid amide surfactants corresponding to the structural formula:

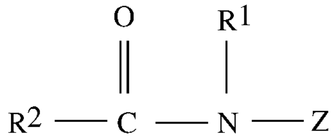

wherein: $R^1$ is H, $C_1$-$C_4$ alkyl, 2-hydroxyethyl, 2-hydroxy- propyl, preferably $C_1$-$C_4$ alkyl, more preferably methyl or ethyl, most preferably methyl; $R^2$ is $C_5$-$C_{31}$ alkyl or alkenyl, preferably $C_7$-$C_{19}$ alkyl or alkenyl, more preferably $C_9$-$C_{17}$ alkyl or alkenyl, most preferably $C_{11}$ -$C_{15}$ alkyl or alkenyl; and Z is a polhydroxyhydrocarbyl moiety having a linear hydrocarbyl

chain with a least 3 hydroxyls directly connected to the chain, or an alkoxylated derivative (preferably ethoxylated or propoxylated) thereof. Z preferably is a sugar moiety selected from the group consisting of glucose, fructose, maltose, lactose, galactose, mannose, xylose, and mixtures thereof. An especially preferred surfactant corresponding to the above structure is coconut alkyl N-methyl glucoside amide (i.e., wherein the $R^2CO$- moiety is derived from coconut oil fatty acids). Processes for making compositions containing polyhydroxy fatty acid amides are disclosed, for example, in G.B. Patent Specification 809,060, published February 18, 1959, by Thomas Hedley & Co., Ltd.; U.S. Patent No. 2,965,576, to E. R. Wilson, issued December 20, 1960; U.S. Patent No. 2,703,798, to A. M. Schwartz, issued March 8, 1955; and U.S. Patent No. 1,985,424, to Piggott, issued December 25, 1934. Preferred among the nonionic surfactants are those selected from the group consisting of steareth-21, ceteareth-20, ceteareth-12, sucrose cocoate, steareth-100, PEG-100 stearate, and mixtures thereof.

Other nonionic surfactants suitable for use herein include sugar esters and polyesters, alkoxylated sugar esters and polyesters, C1-C30 fatty acid esters of C1-C30 fatty alcohols, alkoxylated derivatives of C1-C30 fatty acid esters of C1-C30 fatty alcohols, alkoxylated ethers of C1-C30 fatty alcohols, polyglyceryl esters of C1-C30 fatty acids, C1-C30 esters of polyols, C1-C30 ethers of polyols, alkyl phosphates, polyoxyalkylene fatty ether phosphates, fatty acid amides, acyl lactylates, and mixtures thereof. Nonlimiting examples of these non-silicon-containing emulsifiers include: polyethylene glycol 20 sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate, Polysorbate 60, glyceryl stearate, polyoxyethylene 20 sorbitan trioleate (Polysorbate 85), sorbitan monolaurate, polyoxyethylene 4 lauryl ether sodium stearate, polyglyceryl-4 isostearate, hexyl laurate, PPG-2 methyl glucose ether distearate, PEG-100 stearate, and mixtures thereof.

Fatty Alcohols

The liquid foamable composition may include from 0.1%, 1%, 2%, or 4% to 6%, 7%, 8%, 8% ot 10%, by weight of the liquid foamable composition, of a fatty alcohol. A liquid foamable composition can include a fatty alcohol. For example, the liquid foamable composition can include monohydric saturated straight-chain fatty alcohols, such as one or more of behenyl alcohol, cetyl alcohol, and stearyl alcohol, and other waxy fatty alcohols having melting points of about 25 °C or higher, or of about 45 °C or higher.

In certain examples, the fatty alcohols can be non-volatile and have a low melting point. For example, such fatty alcohols can have a melting point of 30°C or less, about 25°C or less, or about 22°C or less. Unsaturated fatty alcohols can also be non-volatile. Suitable fatty alcohols can include unsaturated monohydric straight-chain fatty alcohols, saturated branched-chain fatty alcohols, saturated $C_8$-$C_{12}$ straight-chain fatty alcohols, and mixtures thereof. The unsaturated straight-chain fatty alcohols can typically have one degree of unsaturation. Di- and tri-unsaturated alkenyl chains can be present at low levels; about 5% or less, by total weight of the unsaturated straight-chain fatty alcohol; about 2% or less, by total weight of the unsaturated straight-chain fatty alcohol; and about 1% or less, by total weight of the unsaturated straight-chain fatty alcohol. The unsaturated straight-chain fatty alcohols can have an aliphatic chain size of from $C_{12}$-$C_{22}$ in certain examples, from $C_{12}$-$C_{18}$ in certain examples, and from $C_{16}$-$C_{18}$ in certain examples. Exemplary alcohols of this type can include oleyl alcohol and palmitoleic alcohol.

Branched-chain alcohols can typically have aliphatic chain sizes of from $C_{12}$-$C_{22}$, $C_{14}$-$C_{20}$ in certain examples, and $C_{16}$-$C_{18}$ in certain examples. Suitable branched-chain alcohols can include isostearyl alcohol, octyl dodecanol, and octyl decanol.

Examples of saturated $C_8$-$C_{12}$ straight-chain alcohols can include octyl alcohol, caprylic alcohol, decyl alcohol, and lauryl alcohol. Fatty alcohols having a low melting point can be included at levels from about 0.1% to about 10%, by weight of the liquid foamable composition, from about 0.2% to about 5%, by weight of the liquid foamable composition in certain examples; and from about 0.5% to about 3%, by weight of the liquid foamable composition in certain examples.

It may be desirable to use waxy fatty alcohols for their conditioning benefits. However, if both waxy fatty alcohols and liquid fatty alcohols are present, a weight ratio of liquid to waxy fatty alcohols can be about 0.25 or less, in certain examples; about 0.15 or less, in certain examples.

In certain examples, a ratio of the fatty alcohol to the surfactant can be about 2 parts to about 1 part. In such examples, the fatty alcohol and the surfactant can combine to form liquid crystal structures in a lamellar gel phase. In examples where the ratio of the fatty alcohol to the surfactant is lower (i.e., an amount of surfactant is increased relative to an amount of fatty alcohol), the liquid crystal structures can be in the form of vesicles. In certain examples, the liquid crystal structures can be of any of a variety of suitable phases including, for example, bicontinuous cubic, hexagonal, inverse cubic, micellar cubic, reverse hexagonal columnar, and combinations thereof. Examples of liquid crystal structures are further described in U.S. Patent No. 8,470,305 and PCT International Publication No. WO 2010/060131.

Other Components

The liquid foamable composition must include water in amount such that water can provide a remainder of the liquid foamable composition, as such, a liquid foamable composition must include from about 50% to about 80%, by weight; or from about 70% to about 75%, by weight, of water.

In certain examples, the water may include other liquid, water-miscible, or water-soluble solvents such as lower alkyl alcohols (e.g., $C_1$-$C_5$ alkyl monohydric alcohols), such as $C_2$-$C_3$ alkyl alcohols. However, the liquid fatty alcohol must be miscible in an aqueous portion of the liquid foamable composition. The fatty alcohol can be naturally miscible in the aqueous portion or can be made miscible through the use of co-solvents or surfactants.

The liquid foamable composition can also include a variety of other optional components suitable for rendering such compositions more cosmetically or aesthetically acceptable or to provide them with additional usage benefits. Such conventional optional ingredients can be well-known to those skilled in the art.

For example, the liquid foamable composition can also include one or more additional conditioning agents, such as those selected from the group consisting of avocado oil, fatty acids, hexyldecanol, isopropyl myristate, lanolin, apple wax, bees wax or jojoba oil, phospholipids (e.g., lecithines or ceramides), vaseline non-volatile hydrocarbons, and hydrocarbon esters. Imidazolidinyl derivatives, such as INCI Quaternium-87 (REWOQUAT® W 575 of Witco, Germany) can also be useful.

In certain examples, the liquid foamable composition can include a superabsorbent polymer. Suitable superabsorbent polymers can include polyacrylates (e.g., sodium polyacrylate starch) and polyacrylic acid polymers. Suitable materials are described, for example, in PCT Patent Applications WO 07/047598, WO 07/046052, WO2009/155265 and WO2009/155264. In certain examples, suitable superabsorbent polymer particles can be obtained by current state-of-the-art production processes, such as those described in WO 2006/083584. The superabsorbent polymers can be internally cross-linked (i.e., polymerization can be carried out in the presence of compounds having two or more polymerizable groups that can be free-radically copolymerized into the polymer network), externally surface crosslinked, or post crosslinked. Additional suitable superabsorbent polymers are described in U.S. Patent Publication No. 2013/0243836 and PCT International Application No. PCT/US2013/032922.

A wide variety of additional ingredients can be included within the liquid foamable composition. Such ingredients can include other conditioning agents (e.g., betaine, carnitin esters, creatine, amino acids, peptides, proteins and vitamins); vitamin derivatives (e.g., tocophenyl actetate, niacinamide, panthenol); hair-hold polymers; detersive surfactants (e.g., anionic, nonionic, amphoteric, and zwitterionic surfactants); UV-filters (e.g., p-methoxy cinnamic acid isoamylester, lipophilic cinnamic acid esters, salicylic acid esters, 4-amino benzoic acid derivatives or hydrophilic sulfonic acid derivatives of benzophenones or 3-benzyliden campher); antioxidants (e.g., tocopheroles), preservatives (e.g., benzyl alcohol, methyl paraben, propyl paraben, and imidazolidinyl urea); polyvinyl alcohol; ethyl alcohol; pH-adjusting agents (e.g., citric acid, formic acid, glyoxylic acid, acetic acid, lactic acid, pyruvic acid, sodium citrate, succinic acid, phosphoric acid, sodium hydroxide, and sodium carbonate); salts (e.g., potassium acetate and sodium chloride); antimicrobials; humectants (e.g., sorbitol); chelators (e.g., such as those described in U.S. Patent No. 5,487,884 issued to Bisset, et al.); sunscreens; desquamation actives (e.g., those described in U.S. Patent No. 5,681,852 and 5,652,228 issued to Bisset); anti-wrinkle/anti-atrophy actives (e.g., N-acetyl derivatives, thiols, hydroxyl acids, phenol); skin soothing agents/skin healing agents (e.g., panthenoic acid derivatives, aloe vera, allantoin); skin lightening agents (e.g., kojic acid, arbutin, ascorbic acid derivatives); skin tanning agents (e.g. dihydroxyacteone); anti-acne medicaments; essential oils; sensates; coloring agents; perfumes; sequestering agents (e.g., disodium ethylenediamine tetraacetate); and polymer plasticizing agents (e.g., glycerin, disobutyl adipate, butyl stearate, and propylene glycol). Other such suitable examples of such skin actives are described in U.S. Patent Application Publication No. 2012/0009285.

Such optional ingredients generally can be used individually at levels from about 0.01% to about 10.0%, by weight of the liquid foamable composition in certain examples; and in certain examples from about 0.05% to about 5.0% of the liquid foamable composition.

In certain examples, the liquid foamable composition can further include one or more thickening agents to facilitate foam stabilization when the propellant is added to the liquid foamable composition. However, in certain examples, the liquid foamable composition can be substantially free of any thickening agents. Non-limiting classes of thickening agents include those selected from carboxylic acid polymers, crosslinked polyacrylate polymers, polyacrylamide polymers, polysaccharides, and gums. Suitable examples of each are described in U.S. Patent Publication No. 2003/0049212. Additionally, suitable thickening agents can include water-soluble polymers as described in U.S. Patent No. 8,444,716. The liquid foamable composition can include from about 0.1% to about 2%, by weight; from about 0.2% to about 1%, by weight; and from about 0.5% to about 1%, by weight, of a polymer thickening agent.

B. Propellant

A non-hydrocarbon propellant is provided for foaming the liquid foamable composition. The cosmetic foam

comprises from 0.1% to 5% of non-hydrocarbon propellant. The non-hydrocarbon propellants are selected from the group consisting of: carbon dioxide and nitrous oxide. In the event that one or both of these propellants are used, alone or in combination, the foam remains stable for a period of time following dispensation, as shown in Inventive Examples 1 and 2 below. By contrast, where a hydrocarbon propellant (such as, for example, dimethyl ester) is used, the foam rapidly destabilizes - as demonstrated with Comparative Example 1 below.

III. Aerosol Product

An aerosol product can include a liquid foamable composition, a propellant, and a package. In certain examples, the liquid foamable composition and propellant can be housed in the package, which can include a container and a valve, such that the liquid foamable composition and propellant can be combined and dispensed as a foam. In certain examples, a foam composition can be housed in a package.

The container can be any of a variety of aerosol containers or similar type containers known in the art. For example, the container can be a single chamber container or a barrier container. Non-limiting examples of single chamber containers can include plastic, glass, aluminum, or steel containers that can be unlined or lined with materials such as epoxy phenolics, organosols, and polyamide imides. In such single chamber containers, the liquid foamable composition and the propellant can be combined in the single chamber, as shown in FIG. 1. Barrier containers can keep the liquid foamable composition physically separate from the propellant within the container. Non-limiting examples of barrier containers can include a piston container and a bag-on-valve container, which are described in U.S. Patent Publication No. 2012/0288465.

The valve can be any of a variety of aerosol valves or similar type valves (e.g., any of a variety of valves supplied by APTAR®). In certain examples, the valve can be a powder valve. The powder valve can include one or more orifices on a valve stem, normally one or two orifices. Each of the orifices can have a same or different orifice diameter and can be in the form of any of a variety of shapes (e.g., circular, square, etc.). Both the orifice diameter and the orifice shape can be selected based upon the size and shape of the particulate material used in the liquid foamable composition. Further, certain valves, such as a powder valve, can help to prevent clogging of the aerosol product by wiping an opening of the orifice against a sealing gasket as the valve moves from an open position to a closed position. Non-limiting examples of suitable powder valve configurations are described in detail in U.S. Patent Nos. 3,773,064, 5,975,378, 6,394,321 and 8,580,725.

FIG. 1 shows a portion of a container 110 to which a valve is mounted, according to one example. A valve assembly 111 can generally include a dip tube 112, a valve housing 114, a valve-closing coil spring 116, and a valve body 118. The valve body 118 can have a hollow valve stem 120 extending upwardly therefrom and can include at least one orifice 122 leading into an interior of the valve stem 120. A sealing gasket 124, which can be made of rubber or other suitable resilient material, can surround the valve stem 120 and seal the orifice 122 when the valve is in the closed position. An actuator 126 having a nozzle 128 is shown to be attached to a top of the valve stem 120. When the actuator 126 is depressed downwardly against a force of the spring 116, the valve moves to the open position, and the orifice 122 can pass below the sealing gasket 124 such that the liquid foamable composition within the container can, under the influence of the propellant, pass up through the dip tube 112, into the valve body 118, through the orifice 122, into the valve stem 120, into the actuator 126, before being dispensed out through the nozzle 128. When the actuator 126 is released, the valve can return to the closed position, such that the spring 116 can push the valve stem 120 and the orifice 122 upwardly against the sealing gasket 124, wiping any remaining liquid foamable composition away from the orifice 122 of the valve stem 120 to prevent clogging of the orifice 122 and blocking flow of the liquid foamable composition.

The actuator 126 can be any of a variety of actuators known in the art. For example, an actuator can be a front-hinged, rear-hinged, or non-hinged actuator, as long as the actuator can be properly matched with the valve stem. Non-limiting examples of suitable hinged actuators can include those available from SEAQUIST® Perfect Dispensing under the trade names S30, S25, S20, and Allegra for upright containers and S16 and S4 for inverted containers. Non-hinged actuators can be used as they can tend to exhibit less lateral pressure during actuation of the aerosol product. Non-limiting examples of suitable non-hinged actuators can include those available from Precision Valve under the trade names City Spout, Hercules Spout, and Iris and those available from SEAQUIST® Perfect Dispensing under the trade name S2. Actuators, valves, containers, and other related parts and equipment can include those available from, for example, APTAR®, Precision Valve, and Summit Packaging Systems.

In another example, a container can include a bag-on-valve system, as mentioned herein and as shown in FIGS. 2 and 3. FIG. 2, for example, shows a bag-on-valve system including a container 210 having an inner bag 213, which can be filled with the foam composition or the liquid foamable composition, and an outer container 215, which can enclose the inner bag 213. A valve assembly 211, vertically movable between an open position and a closed position, can be attached to the inner bag 213.

The valve assembly 211 can include a housing 214, a valve stem 220, a spring 216, a valve plate 232, an inner sealing 234, and an outer sealing 236. The valve stem 220 can include one or more lateral openings 238. The spring

216 can be disposed between a lower end portion 240 of the valve stem 220 and the housing 214 and can bias the valve stem 220 upwardly towards the valve plate 232, which can be disposed at an upper end of the housing 214. The valve plate 232 can include two coaxially-arranged recesses 242, 244 extending in a circumferential direction of the valve plate 32. FIG. 2 shows an axial opening 246 located in a central portion of the inner recess 242. The inner sealing 234 can be disposed within the inner recess 242, attached to the valve plate 232, and can be adapted to engage the valve stem 220 such that the lateral opening 238 of the valve stem 220 is covered and blocked, respectively. The outer sealing 236 can be disposed in the second or outer recess 244 of the valve plate 232. The valve stem 220 can include a passage 248 in the central axial portion thereof, which can be connected to the lateral opening 238 on one side and connectable to a corresponding passage of a dispenser cap on the other side. In the closed position, a flow path from the interior space of the housing 214 along the valve stem 220 and through the lateral opening 238 can be blocked by the inner sealing 234.

The valve assembly 211 can be fixed to the inner bag 213 at an upper end thereof such that a lower end of the housing 214 of the valve assembly 211 can be gas-tight covered by the upper edge of the inner bag 213. Further, the inner bag 213 and the valve assembly 211 can be attached to the outer container 215 such that an upper end of the outer container 215 can engage the outer sealing 236 of the valve plate 232 in a gas-tight manner. Accordingly, an interior of the inner bag 213 and space between the outer container 215 and the inner bag 213 each can be independently sealed.

A dispenser cap having an actuator (not shown) can be attached to the valve plate 232 such that the actuator can engage the valve stem 220. When the actuator is depressed downwardly against a force of the spring 216, the valve assembly 211 can move to the open position. The valve stem 220 moves within the inner sealing 234, which can remain stationary, while contacting the same. Once the lateral opening 238 can be uncovered by the inner sealing 234, the flow path from the valve housing 214 through the lateral opening 238 can be opened. Thus, the interior of the inner bag 213 and the flow path inside the valve housing 214 become linked such that the foam composition/liquid foamable composition within the inner bag 213 can pass through the flow path and dispensed out of the dispenser cap by the pressure of the propellant/compressed gas, which can surround the inner bag 213.

As shown in FIG. 3, the inner bag 213 can include flat lateral edges 250 and a bottom fold 252, which can be directed towards an upper end of the inner bag 213 in order to allow a controlled collapse. Near the bottom fold 252, the inner bag 213 can include two flat triangular portions 254, each extending from the bottom edge 256 to the lateral edge 250 with an angle of about 45°. This can further facilitate the collapse of the inner bag 213, when compressed by the pressure of the propellant in the outer container 215 (as shown in FIG. 2). As described above, the outer container 215 can include any of a variety of propellants or any other suitable compressed gas. Pressure of the propellant can be set to from about 0.3 to about 1.0 MPa, or from about 0.3 to about 0.8 MPa, in order to stably discharge contents of the inner bag 213 as completely as possible.

The inner bag can be flexible, and can be made from any of a variety of suitable materials. In certain examples, the inner bag can be formed with a layer of a material that can be essentially impermeable to the propellant within the inner bag. In certain examples, the inner bag can be formed with a layer of a material that can be essentially impermeable to the propellant outside of the bag, as it may be required that such compositions do not mix during storage. Mixing of the propellant within the inner bag and the propellant outside of the bag can be inappropriate based on the properties of the foam composition/liquid foamable composition or any of a variety of other reasons. However, this does not preclude the possibility that the propellant within the inner bag and the propellant outside of the bag can be mixed upon dispensing of the foam composition/liquid foamable composition when a valve to dispense the foam is triggered. For example, a mixing channel (not shown) or another appropriate measure can be used in such a case to mix the respective propellants if desired.

IV. Method of Use

The foam composition can be used in conventional ways to improve sensory benefits to skin. This generally involves application of an effective amount of the foam composition to a portion of the skin of a user. For example, the foam composition can be dispensed from an aerosol can or similar container or package, and the foam composition can be applied and rubbed onto a desired portion of the skin of a user. An "effective amount" can refer to an amount sufficient enough to provide the desired sensory benefits, which can include, for example, a rich and creamy appearance and a favorable "feel."

In certain embodiments, the foam composition can provide the rich and creamy appearance and moisturization and protection capabilities associated with heavier products, while providing a rapid absorption and ease of application associated with lighter products. Furthermore, the foam composition can reduce or eliminate characteristics associated with a negative sensory experience such as, for example, tack, drag, and stickiness.

V. Procedures

Polydispersity Index

Polydispersity Index (PDI) is calculated as the standard deviation of the mean bubble area divided by the mean bubble area (SDMBA/MBA). These values are obtained by conducting optical foam structure analysis, using the Kruss Dynamic Foam Analyzer - DFA100FSM and Kruss Advance software, as follows:

1) Prepare Kruss Dynamic Foam Analyzer measuring cylinder (with prism) by creating a platform inside the cylinder, such that only the top 1" of the cylinder is open for filling with foam; Position the cylinder into the Dynamic Foam Analyzer.
2) In Kruss Advance software, use the Foam Structure Module (FSM) to start video capture at rate of 2 frames/second.
3) Dispense foaming product from a pressurized dispenser containing a non-hydrocarbon propellant into a measuring cylinder, filling until the top of the cylinder is full of foam or until the video frame is filled with foam.
4) After the desired time is reached, stop the video capture.
5) Record MBA and SDMBA and calculate PDI at 5 seconds and 60 seconds after dispensing

a. A time correction on the video frames must take into account the time delay between the time that the video capture started (step 2 above) and the time of first frame of foam is observed. The first frame in which foam is observed is considered time 0s. The 5 second and 60 second readings are taken from this start time.

Foam Density Determination

From a pressurized dispenser containing the foam composition, dispense enough foam into a small cylindrical cup-like container of known volume (or dimensions) and weight, such that the foam composition can rise above a rim of the cup-like container. Using a tool with a straight edge, such as a spatula, scrape off any excess foam by sweeping an edge of the spatula across the rim of the cup-like container to leave a flat smooth surface at level with a top of the cup-like container. Weigh the foam composition and the container, and calculate a foam density using the following formula:

$$foam\ density = \frac{weight\ of\ cup\ with\ foam\ (g) - weight\ of\ empty\ cup(g)}{Volume\ of\ Cup\ (mL)}$$

Assuming the cup-like container is cylindrical, the volume of the container can be calculated by measuring its diameter and depth with, for example, a caliper or similar measuring tool. The volume can then be calculated using the following formula:

$$Volume = (\pi)\times(cup\ height\ [mm])\times\left(\frac{cup\ diameter\ [mm]}{2}\right)^2$$

VI. Examples

A. Inventive Example

Each of the inventive examples can be prepared by combining the water, cationic surfactant, and fatty alcohol and heating the mixture to about 80°C. Liquid crystal structures (e.g., a lamellar gel structure) can be formed as the quaternary ammonium compound and the fatty alcohol combine. The mixture can then be allowed to cool, at which point the crystal structures thicken the water phase. Subsequently, the remaining components can be added to the cooled lamellar gel. Comparative Example 1 and Inventive Examples 1 and 2 are all formed with the same liquid foamable composition, comprising a cationic and/or nonionic surfactant and a fatty alcohol. Comparative Example 1, shown in FIGs. 4(a) and 4(b), shows qualitatively the foam bubbles generated when using a hydrocarbon propellant, specifically dimethyl ether at 5 seconds post dispensation (FIG. 4(a)) and at 60 seconds post dispensation (FIG. 4(b)). By contrast, FIGs. 5 and 6 show clearly the finer bubbles seen from foam dispensed using a nonhydrocarbon propellant. Specifically, FIGs. 5(a) and (b) show the liquid foamable composition when foamed with carbon dioxide and FIGs. 6(a) and (b) show the liquid foamable composition when foamed with nitrous oxide. Table 1 below shows an expert evaluation of the different foams at 60 seconds post dispensation, from which it can clearly be seen that Inventive Examples 1 and 2 were

preferred. Furthermore, FIG. 7 is a graph showing the PDI for the different examples as time progresses. From this, it can be seen clearly that the foams formed with a non-hydrocarbon propellant (Inventive Examples 1 and 2) have a fairly consistent and less than 2.5 PDI as time progresses, reflecting a stability in structure of the foam.

TABLE 1

| **Evaluation at 60 sec.** | **Foam (DME at 60 s)** | **Foam (C02 at 60 s)** | **Foam (N2O at 60s)** |
|---|---|---|---|
| Overall | poor | good | good |
| Feel | thin, runny | rich, creamy | rich, creamy |
| Foam quality | poor | good | good |
| Visual | semi translucent | opaque | opaque |
| Bubble size | very coarse, large voids | smooth, homogenous | smooth, homogenous |
| Stability | poor | good | good |

Table 2 sets out the ingredient list for the liquid foamable composition in the comparative and inventive examples.

TABLE 2

| Materials | Comp. Ex. 1 | Inv. Ex. 1 | Inv. Ex. 2 |
|---|---|---|---|
| DC9040 (Cyclopentasiloxane (and) Dimethicone Crosspolymer) | 8.1 | 8.1 | 8.1 |
| KSG-16F (Dimethicone (and) Dimethicone/Vinyl Dimethicone Crosspolymer) | 3.3 | 3.3 | 3.3 |
| Cyclopentasiloxane Fluid | 3.6 | 3.6 | 3.6 |
| Water | 72.4 | 74.4 | 74.4 |
| Behenyl Trimethyl Ammonium Chloride | 1.4 | 1.4 | 1.4 |
| Cetyl Alcohol | 0.9 | 0.9 | 0.9 |
| Stearyl Alcohol | 2.3 | 2.3 | 2.3 |
| SF-1288 (PEG-12 Dimethicone) | 0.50 | 0.50 | 0.50 |
| DC9506 (Dimethicone/Vinyl Dimethicone Crosspolymer) | 3.0 | 3.0 | 3.0 |
| KSP-105 (Vinyldimethicone/methicone silsesquioxane crospolymer) | 1.5 | 1.5 | 1.5 |
| Dimethyl Ether | 3.0 | - | - |
| Carbon Dioxide | - | 1.0 | - |
| Nitrous Oxide | - | - | 1.0 |
| Total | 100 | 100 | 100 |

**Claims**

**1.** A method of use comprising the application of an effective amount of a cosmetic foam composition comprising:

from 0.1% to 5%, by weight of the cosmetic foam composition, of a non-hydrocarbon propellant; and said propellant is selected from the group consisting of: carbon dioxide, nitrous oxide and a combination thereof;
a liquid foamable composition comprising 0.05% to 5%, by weight of the liquid foamable composition, of a surfactant selected from the group consisting of a cationic surfactant, nonionic surfactant, and mixtures thereof, and 1% to 10%, by weight of the liquid foamable composition, of a fatty alcohol, wherein the liquid foamable composition comprises 50% to 80%, by weight of the liquid foamable composition, of water to a portion of the skin of a user, with the exception of liquid foamable compositions comprising from 0.05% to 5%, by weight, of surfactants selected from the group consisting of cationic surfactants, non-ionic surfactants, and mixtures thereof, from 1% to 10%, by weight of a fatty alcohol, from 10% to 50%, by weight, of a silicone blend, the silicone blend comprising a first silicone component comprising a silicone gel; and a second silicone component comprising a silicone powder; and water.

2. A method as claimed in claim 1, wherein the liquid foamable composition is 95% to 99.9% by weight of the cosmetic foam composition.

3. A method as claimed in any preceding claim, wherein the cosmetic foam composition comprises from 0.25% to 2% of non-hydrocarbon propellant.

4. A method as claimed in any preceding claim, wherein the fatty alcohol has a melting point of about 25°C or higher, and preferably the fatty alcohol comprises one or more of behenyl alcohol, cetyl alcohol, and stearyl alcohol.

5. A method as claimed in any preceding claim, wherein the surfactant comprises a quaternary ammonium compound, preferably the quaternary ammonium compound comprises one or more of behenyl trimethyl ammonium chloride, stearamidopropyl dimethylamine, behentrimonium methosulfate, behenylamidopropyl dimethylamine, stearyl ethylhexyldimonium methosulfate, dicetyldimonium chloride, and ditallow dimethyl ammonium chloride.

**Patentansprüche**

1. Verfahren zur Verwendung, umfassend das Auftragen einer wirksamen Menge einer kosmetischen Schaumzusammensetzung, umfassend:

   zu von 0,1 Gew.-% bis 5 Gew.-% der kosmetischen Schaumzusammensetzung ein kohlenwasserstofffreies Treibmittel; und wobei das Treibmittel ausgewählt ist aus der Gruppe bestehend aus: Kohlendioxid, Stickstoffoxid und einer Kombination davon;
   eine flüssige schäumbare Zusammensetzung, umfassend zu 0,05 Gew.-% bis 5 Gew.-% der flüssigen schäumbaren Zusammensetzung ein Tensid ausgewählt aus der Gruppe bestehend aus einem kationischen Tensid, nichtionischen Tensid und Mischungen davon, und zu 1 Gew.-% bis 10 Gew.-% der flüssigen schäumbaren Zusammensetzung einen Fettalkohol, wobei die flüssige schäumbare Zusammensetzung zu 50 Gew.-% bis 80 Gew.-% der flüssigen schäumbaren Zusammensetzung Wasser auf einen Teil der Haut eines Benutzers umfasst, mit Ausnahme von flüssigen schäumbaren Zusammensetzungen, umfassend zu von 0,05 Gew.-% bis 5 Gew.-% Tenside ausgewählt aus der Gruppe bestehend aus kationischen Tensiden, nichtionischen Tensiden und Mischungen davon, zu von 1 Gew.-% bis 10 Gew.-% einen Fettalkohol, zu von 10 Gew.-% bis 50 Gew.-% eine Silikonmischung, wobei die Silikonmischung einen ersten Silikonbestandteil umfassend ein Silikongel umfasst; und einen zweiten Silikonbestandteil, der ein Silikonpulver umfasst; und Wasser;

2. Verfahren nach Anspruch 1, wobei die flüssige schäumbare Zusammensetzung zu 95 bis 99,9 Gew.-% der kosmetischen Schaumzusammensetzung ausmacht.

3. Verfahren nach einem der vorstehenden Ansprüche, wobei die kosmetische Schaumzusammensetzung zu von 0,25 % bis 2 % kohlenwasserstofffreies Treibmittel umfasst.

4. Verfahren nach einem der vorstehenden Ansprüche, wobei der Fettalkohol einen Schmelzpunkt von etwa 25 °C oder höher aufweist und der Fettalkohol vorzugsweise eines oder mehrere von Behenylalkohol, Cetylalkohol und Stearylalkohol umfasst.

5. Verfahren nach einem der vorstehenden Ansprüche, wobei das Tensid eine quartäre Ammoniumverbindung umfasst, wobei die quartäre Ammoniumverbindung vorzugsweise eines oder mehrere von Behenyltrimethylammoniumchlorid, Stearamidopropyldimethylamin, Behentrimoniummethosulfat, Behenylamidopropyldimethylamin, Stearylethylhexyldimoniummethosulfat, Dicetyldimoniumchlorid und Ditalgdimethylammoniumchlorid umfasst.

**Revendications**

1. Procédé d'utilisation comprenant l'application d'une quantité efficace d'une composition de mousse cosmétique comprenant :

   de 0,1 % à 5 %, en poids de la composition de mousse cosmétique, d'un propulseur non hydrocarboné ; et ledit

propulseur est choisi dans le groupe constitué de : dioxyde de carbone, oxyde nitreux et une combinaison de ceux-ci ;
une composition moussante liquide comprenant 0,05 % à 5 %, en poids de la composition moussante liquide, d'un agent tensioactif choisi dans le groupe constitué d'un agent tensioactif cationique, d'un agent tensioactif non ionique, et de mélanges de ceux-ci, et 1 % à 10 %, en poids de la composition moussante liquide, d'un alcool gras, dans lequel la composition moussante liquide comprend 50 % à 80 %, en poids de la composition moussante liquide, d'eau sur une partie de la peau d'un utilisateur, à l'exception de compositions moussantes liquides comprenant de 0,05 % à 5 %, en poids, d'agents tensioactifs choisis dans le groupe constitué d'agents tensioactifs cationiques, agents tensioactifs non ioniques, et des mélanges de ceux-ci, de 1 % à 10 %, en poids d'un alcool gras, de 10 % à 50 %, en poids, d'un mélange de silicones, le mélange de silicones comprenant un premier composant de silicone comprenant un gel de silicone ; et un deuxième composant de silicone comprenant une poudre de silicone ; et de l'eau.

**2.** Procédé tel que revendiqué dans la revendication 1, dans lequel la composition moussante liquide représente 95 % à 99,9 % en poids de la composition de mousse cosmétique.

**3.** Procédé tel que revendiqué dans l'une quelconque revendication précédente, dans lequel la composition de mousse cosmétique comprend de 0,25 % à 2 % du propulseur non hydrocarboné.

**4.** Procédé tel que revendiqué dans l'une quelconque revendication précédente, dans lequel l'alcool gras a un point de fusion d'environ 25 °C ou plus, et de préférence l'alcool gras comprend un ou plusieurs parmi alcool béhénylique, alcool cétylique, et alcool stéarylique.

**5.** Procédé tel que revendiqué dans l'une quelconque revendication précédente, dans lequel l'agent tensioactif comprend un composé d'ammonium quaternaire, de préférence le composé d'ammonium quaternaire comprend un ou plusieurs parmi chlorure de béhényltriméthylammonium, stéaramidopropyl-diméthylamine, méthosulfate de béhéntrimonium, béhénylamidopropyl-diméthylamine, méthosulfate de stéaryl-éthylhexyldimonium, chlorure de dicétyldimonium, et chlorure de di-suif diméthylammonium.

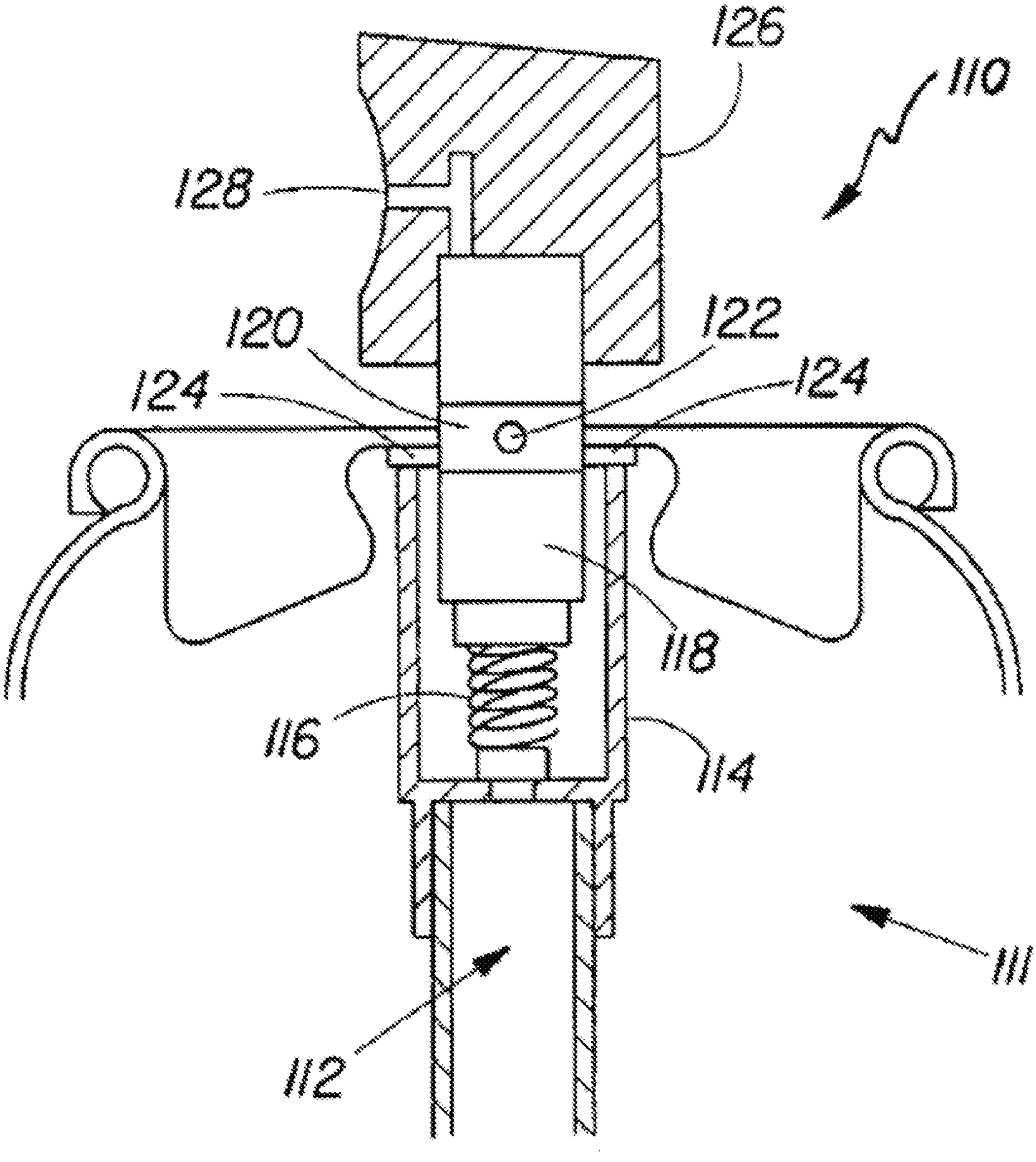


FIG. 1

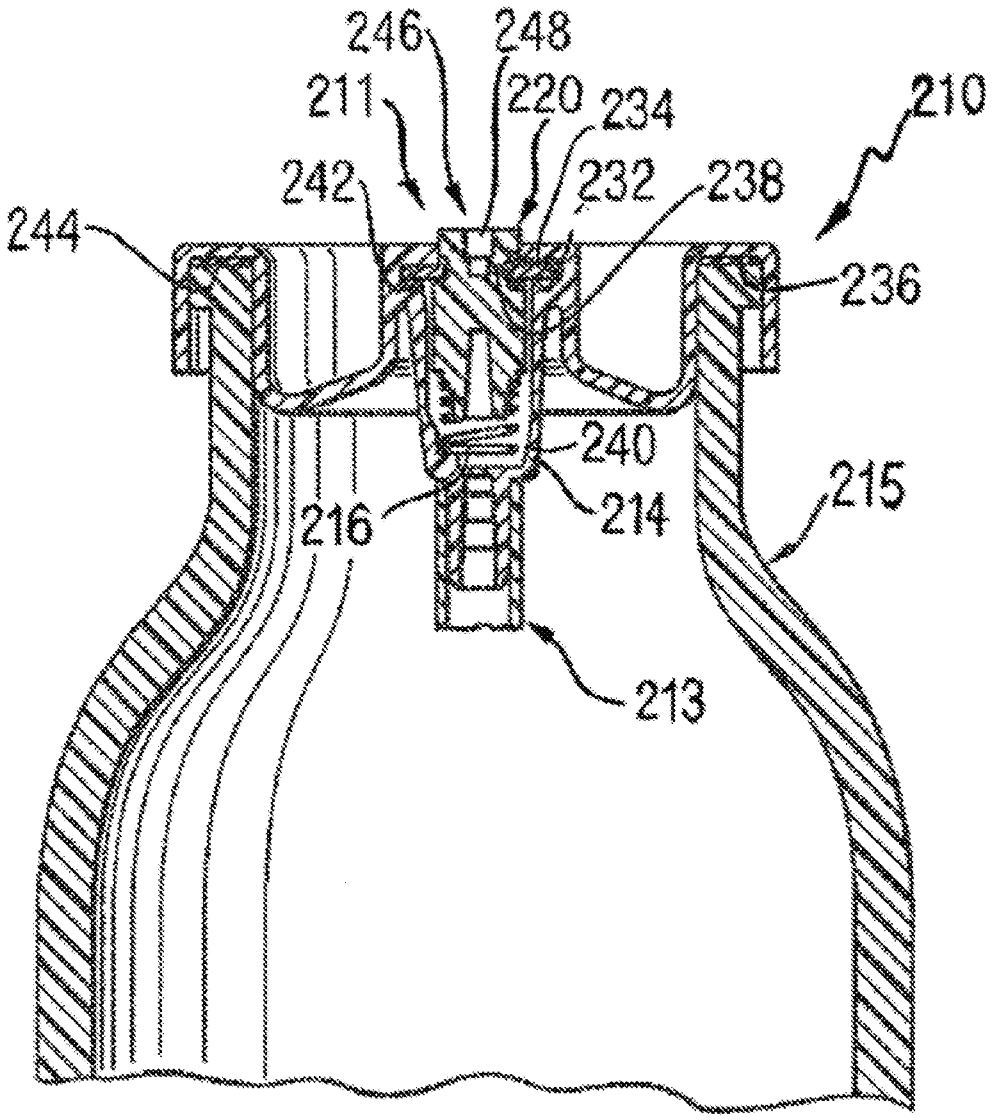


FIG. 2

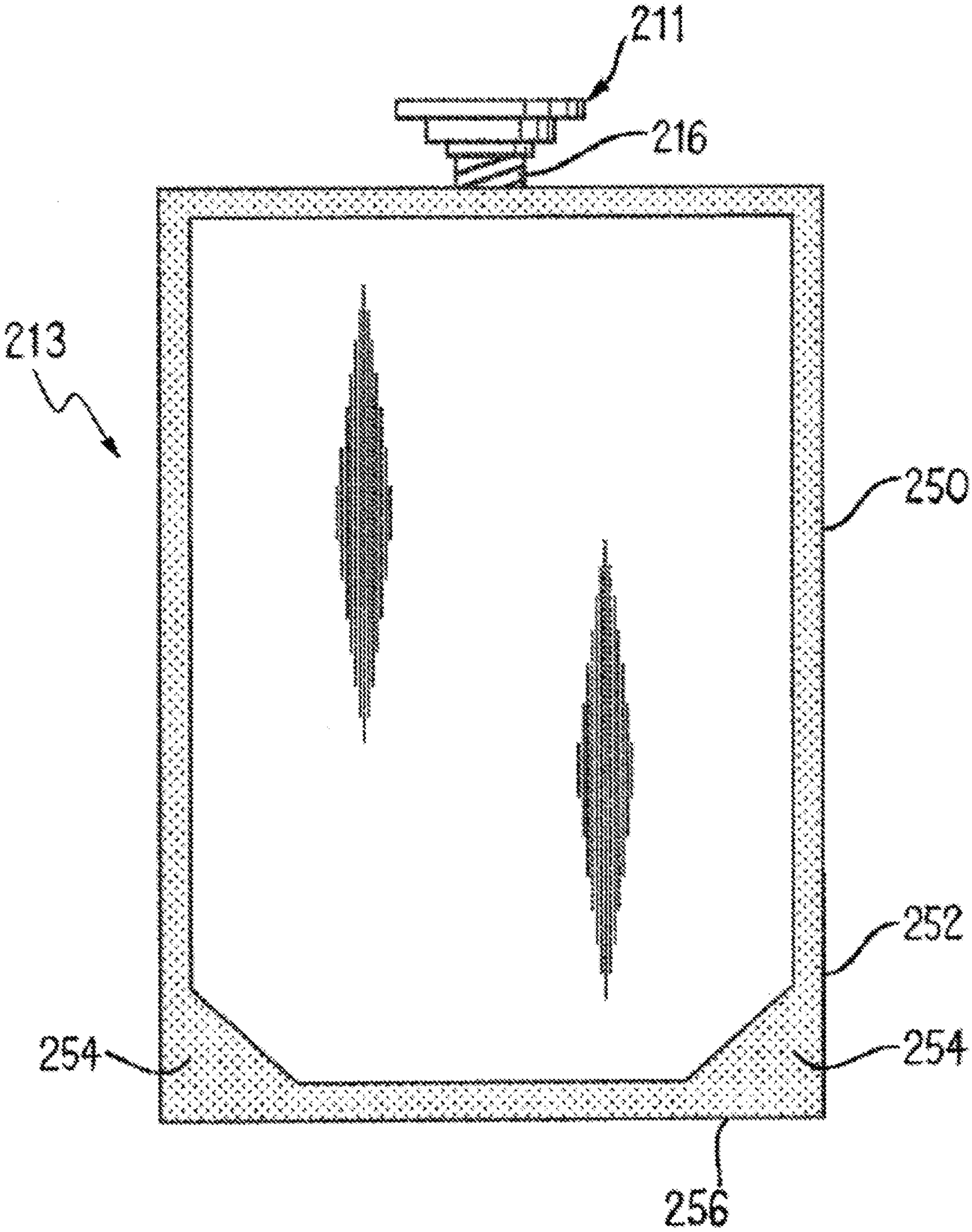


FIG. 3

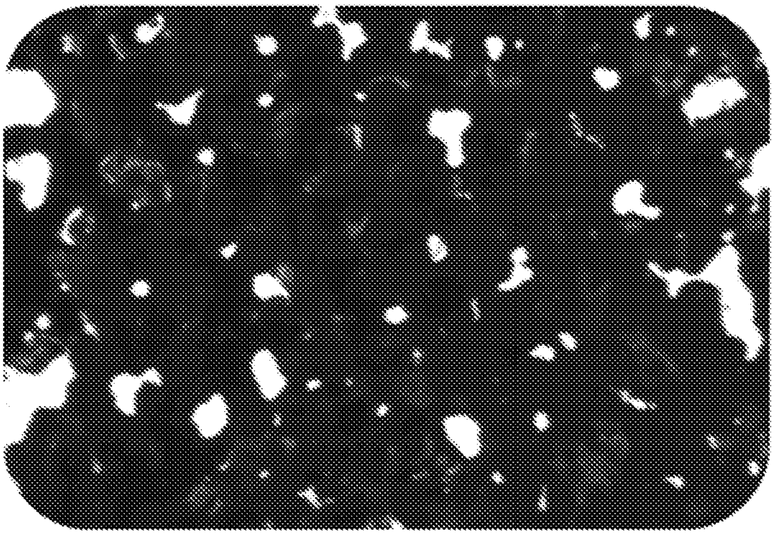

FIG. 4(a)

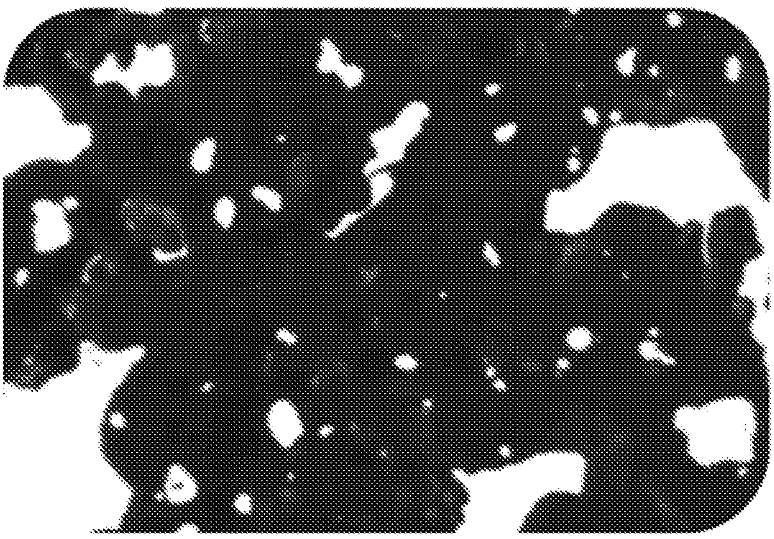

FIG. 4(b)

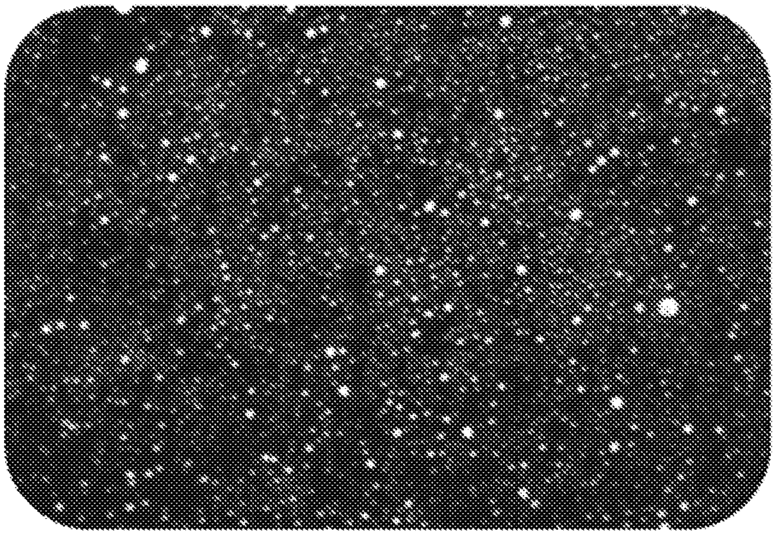

FIG. 5(a)

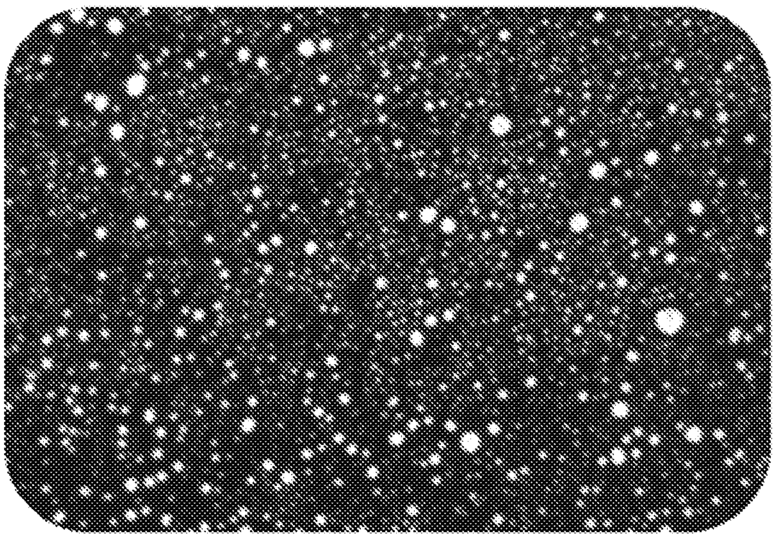

FIG. 5(b)

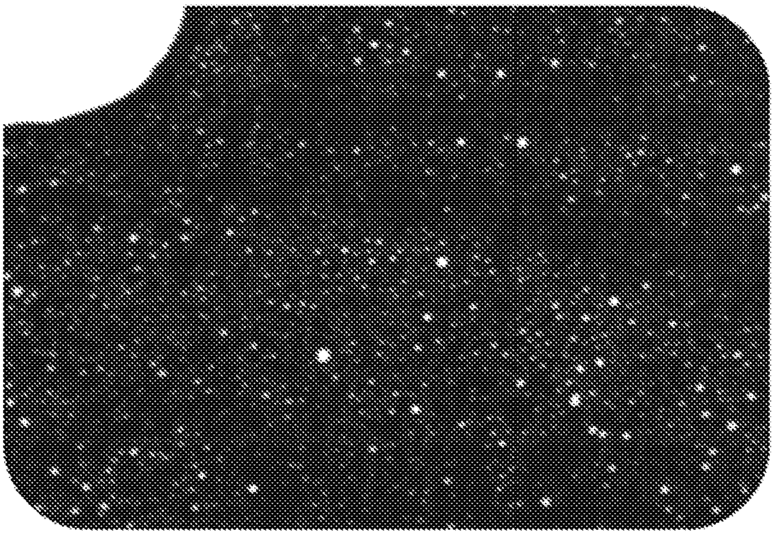

FIG. 6(a)

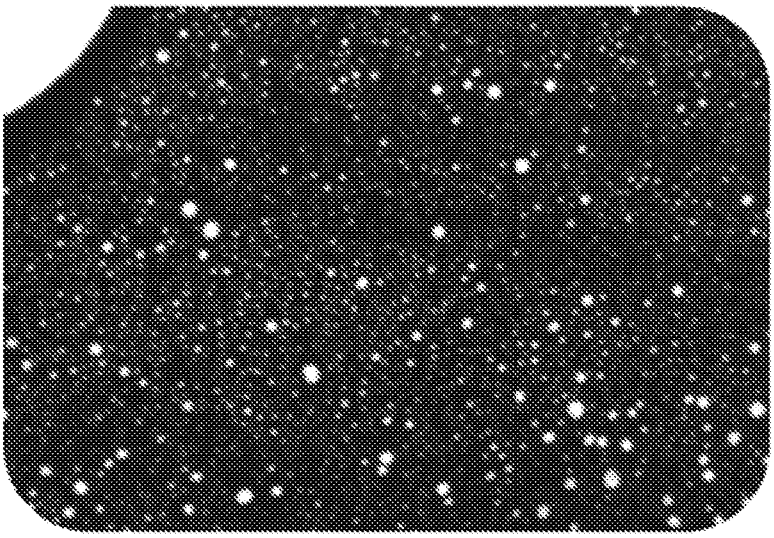

FIG. 6(b)

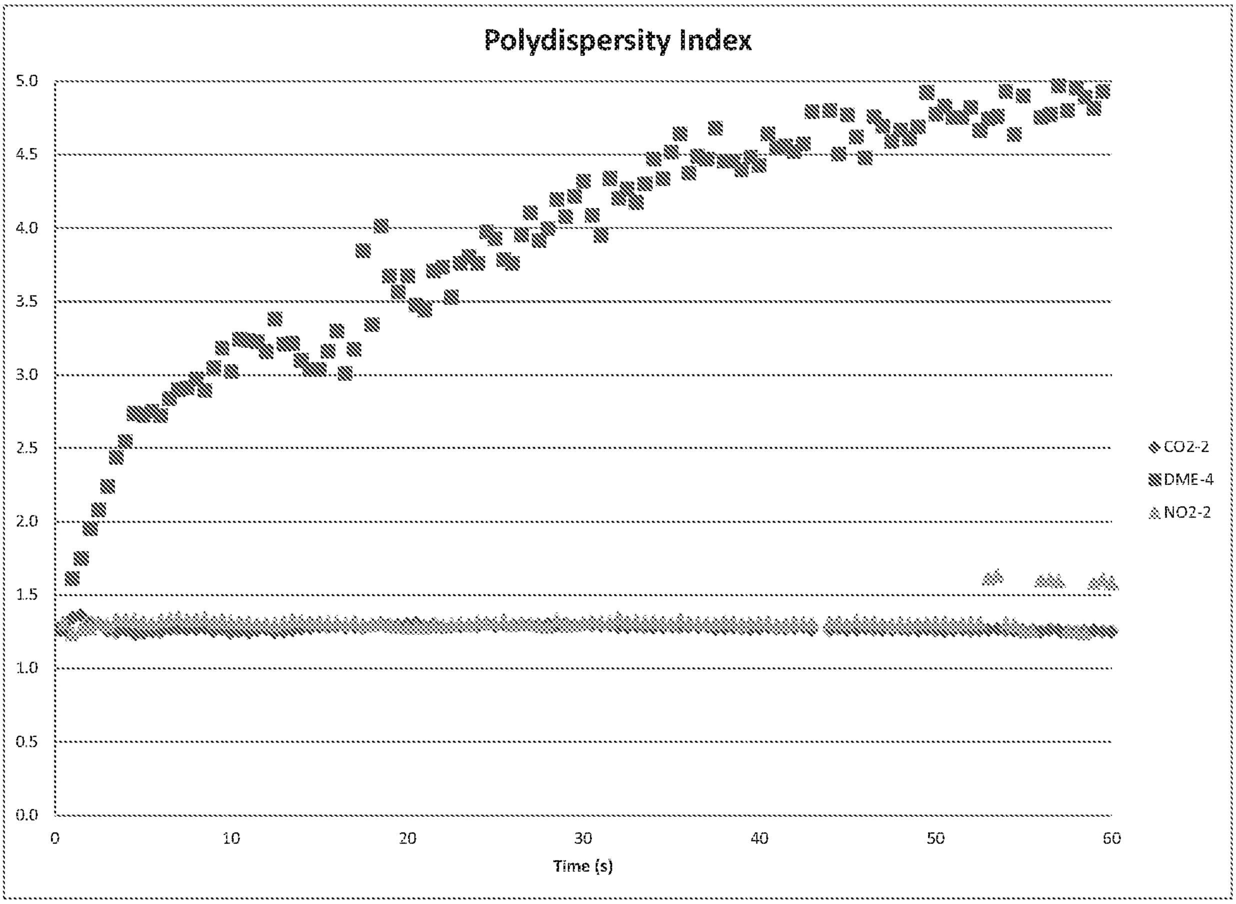
Polydispersity Index
5.0
4.5
4.0
3.5
3.0
2.5
2.0
1.5
1.0
0.5
0.0
0
10
20
30
40
50
60
Time (s)
CO2-2
DME-4
NO2-2

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2003053961 A1
- US 3155591 A, Hilfer
- US 3929678 A, Laughlin
- US 3959461 A, Bailey
- US 4387090 A, Bolich, Jr.
- US 8936798 B
- US 4275055 A, Nachtigal
- GB 809060 A
- US 2965576 A, E. R. Wilson
- US 2703798 A, A. M. Schwartz
- US 1985424 A, Piggott
- US 8470305 B
- WO 2010060131 A
- WO 07047598 A
- WO 07046052 A
- WO 2009155265 A
- WO 2009155264 A
- WO 2006083584 A
- US 20130243836 A
- US 2013032922 W
- US 5487884 A, Bisset
- US 5681852 A
- US 5652228 A, Bisset
- US 20120009285
- US 20030049212 A
- US 8444716 B
- US 20120288465 A
- US 3773064 A
- US 5975378 A
- US 6394321 B
- US 8580725 B


### Non-patent literature cited in the description


- McCutcheon's, Detergents & Emulsifiers. M.C. Publishing Co, 1979
- **SCHWARTZ et al.** Surface Active Agents, Their Chemistry and Technology. Interscience Publishers, 1949